# EUROPÄISCHE PATENTSCHRIFT

(11) **EP 2 622 003 B1**
(45) Veröffentlichungstag und Bekanntmachung des Hinweises auf die Patenterteilung: **26.11.2014**
(21) Anmeldenummer: 11761048.5
(22) Anmeldetag: 19.09.2011
(51) Int. Cl.: A61Q 5/02, A61Q 5/12, A61K 8/06, C08G 77/26, C08G 77/16, C08G 77/388, A61K 8/898, C08L 83/08

(54) **VOLLSTÄNDIG ACYLIERTE AMINOFUNKTIONELLE ORGANOPOLYSILOXANE**
FULLY ACYLATED AMINO-FUNCTIONAL ORGANOPOLYSILOXANES
ORGANOPOLYSILOXANES AMINOFONCTIONNELS TOTALEMENT ACYLÉS

(30) Priorität: 28.09.2010 DE 102010041503
(43) Veröffentlichungstag der Anmeldung: 07.08.2013
(73) Patentinhaber: Wacker Chemie AG, 81737 München (DE)
(72) Erfinder: BREHM, Christof, 84489 Burghausen (DE); BEER, Gerhard, 84489 Burghausen (DE); MERGET, Markus, 84561 Mehring (DE)
(74) Vertreter: Fritz, Helmut
(86) Internationale Anmeldenummer: PCT/EP2011/066201
(87) Internationale Veröffentlichungsnummer: WO 2012/041733

(56) Entgegenhaltungen:
- EP-A2- 0 161 888
- WO-A1-01/23394
- DE-A1- 4 211 269
- JP-A- 11 322 937
- US-A- 4 848 981

## Beschreibung

Die Erfindung betrifft vollständig acylierte aminofunktionelle und gleichzeitig Hydroxy-terminierte Organopolysiloxane, wässrige Emulsionen enthaltend die Organopolysiloxane, deren Verwendung im Bereich Kosmetik und Herstellung der Organopolysiloxane,

Aminogruppen aufweisende Organopolysiloxane, deren Aminogruppen acyliert sind, sind schon seit einiger Zeit Stand der Technik und werden zum Beispiel als Textilausrüstungsmittel eingesetzt. Die Acylierung dient hier vor allem einer wirksamen Reduzierung der Thermovergilbung.

Beispielsweise beschreibt JP-A-57/101076 die teilweise oder vollständige Acetylierung mit Essigsäureanhydrid von aminofunktionalisierten Organopolysiloxanen mit mindestens zwei Amino-Gruppen bei erhöhten Temperaturen von 100 bis 110°C. In EP-A-161 888 werden ebenfalls vollständig acetylierte aminofunktionalisierte Organopolysiloxane - mit zwei Amino-Gruppen in der funktionalisierten Seitenkette - beschrieben. Auch hier wird die Acetylierung mit Essigsäureanhydrid bei höheren Temperaturen (> 68°C) durchgeführt.

Die Viskositäten der Öle steigen durch die vollständige Acylierung in gewissem Maße an. Bei Verwendung von organischen Anhydriden, wie zum Beispiel Essigsäureanhydrid als Acylierungsmittel bilden sich Säuren, wie zum Beispiel Essigsäure, die entweder nicht umgesetzte Aminogruppen protonieren oder aber als freie Säure vorliegen. In beiden Fällen und vor allem bei den erhöhten Temperaturen, wie in den vorliegenden Beispielen beschrieben, wird bei Organosiloxanen mit reaktiven Endgruppen (z.B. Si-OH oder Si-O-Alkyl) die Kondensation katalysiert, was zu einem gegebenenfalls nicht mehr beherrschbaren Viskositätsanstieg bzw. zu Vergelung führt.

Für Anwendungen in der Kosmetik besteht ein Bedarf an öligen, vollständig acylierte Amino-Gruppen aufweisenden Organopolysiloxanen, die gleichzeitig hydrophile Kettenenden aufweisen.

Die Herstellung linearer Hydroxy-terminierter acylierte Amino-Gruppen aufweisender Organopolysiloxane mit Carbonsäureanhydrid in wässriger Emulsion ist in DE-A-4211269 beschrieben. Die Amino-Gruppen werden aber bei diesem Verfahren nicht vollständig acyliert.

Gegenstand der Erfindung sind Hydroxy-terminierte Organopolysiloxane (O) der allgemeinen Formel (1), 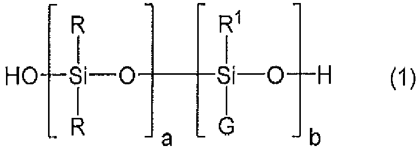 wobei
- **R**: einen einwertigen unsubstituierten oder halogensubstituierten Kohlenwasserstoffrest mit 1 bis 20 Kohlenstoffatomen,
- **R¹**: einen Rest **R,** -O**R⁴** oder -OH,
- **R⁴**: einen Alkylrest mit 1 bis 6 Kohlenstoffatomen,
- **G**: eine Gruppe der allgemeinen Formel (2), 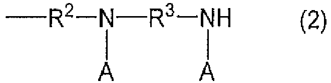
wobei
- **R²**, **R³**: einen zweiwertigen Kohlenwasserstoffrest mit 1, bis 6 C-Atomen, wobei nicht benachbarte -CH₂-Einheiten ersetzt sein können durch Einheiten, die ausgewählt werden aus -C(=O)-, -O-, und -S-,
- **A**: **R⁵**-C(=O)-,
- **R⁵**: Alkylrest mit 1 bis 20 Kohlenstoffatomen,
- **a**: ganzzahlige Werte von 100 bis 1500 und
- **b**: ganzzahlige Werte von mindestens 1 bedeuten.

Es wurden jetzt Verfahren bereitgestellt, die überraschenderweise den Viskositätsanstieg der Hydroxy-terminierten Organopolysiloxane (O) während des Acylierungsschritts beherrschbar und reproduzierbar gestalten und so die Herstellung Hydroxy-terminierten, vollständig acylierten Organopolysiloxane (0) ermöglichen. Ferner sind die erhaltenen Organopolysiloxane (O) viskositätsstabil.

Gegenstand der Erfindung sind weiterhin wässrige Emulsionen (W) enthaltend 5 Gew.-% bis 70 Gew.-% der Hydroxy-terminierten Organopolysiloxane (0) der allgemeinen Formel (1) sowie 1 bis 150 Gew.% bezogen auf das Gewicht des Organopolysiloxanes (O) eines Emulgators (E).

Die Emulsionen (W) zeichnen sich dabei durch eine sehr gute Lagerstabilität wie auch Verdünnungsstabilität aus. Die Emulsionen (W) sind sehr gut in Zusammensetzungen für die Kosmetik wie Shampoo oder Conditioner einsetzbar.

Ebenfalls Gegenstand der Erfindung ist die Verwendung der Emulsionen (W) in Zusammensetzungen für die Kosmetik.

Bereiche der Kosmetik, in denen die Emulsionen (W) bevorzugt eingesetzt werden sind Haarreinigung und -pflege, beispielsweise Shampoos, Conditioner, Pflegespülungen, Haarmasken, Haarfärbeprodukte, Stylingprodukte wie Mousse und Haarbehandlungen wie Dauerwelle und Body Wash, wie Duschbad und Seifen.

Die Emulsionen (W) bewirken im Haar durch ihren Gehalt an Organopolysiloxanen (0) beispielsweise gute Pflegeeigenschaften, guter Weichgriff, Erhöhung des Volumens, Anti-Frizz, Verringerung des Fly-Away, Verringerung der Kämmkraft, Hitzeschutz und Farbschutz.

Ebenfalls Gegenstand der Erfindung ist ein Verfahren A zur Herstellung der Hydroxy-terminierten Organopolysiloxane (O), bei dem Hydroxy-terminierte Organopolysiloxane (N) der allgemeinen Formel (1a) 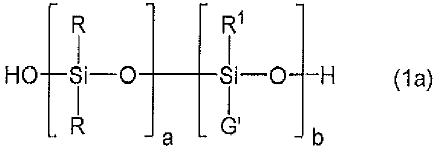 in der
- **G'**: eine Gruppe der allgemeinen Formel (2a) bedeutet 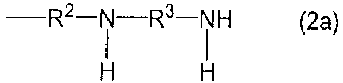
und **R, R¹, R², R³, R⁴, a** und **b** die vorstehenden Bedeutungen aufweisen,
mit acyclischen Carbonsäureanhydriden der allgemeinen Formel (3)

R-⁵-C(=O)-O-C(=O)-R⁵ (3)

, in stöchiometrisch äquivalenter Menge zu den im Organopolysiloxan (N) vorhandenen Amingruppen in Gegenwart von einem oder verschiedenen Emulgatoren (E1) umgesetzt wird.

Überraschenderweise kann durch die Verwendung der Emulgatoren (E1) die Viskosität der Reaktionsmischung während der Acylierung sehr gut kontrolliert werden.

Nach erfolgter Umsetzung kann die Mischung aus Emulgatoren (E1) und Organopolysiloxan (O) sofort emulgiert werden, so dass die erhaltene Ölviskosität von Organopolysiloxan (O) stabil bleibt. Durch die Acylierung zu Organopolysiloxan (O) und dessen Emulgieren in einem Schritt ist eine sehr effiziente Verfahrensführung möglich.

Ebenfalls Gegenstand der Erfindung ist ein Verfahren B zur Herstellung der Hydroxy-terminierten Organopolysiloxane (O), bei dem Hydroxy-terminierte Organopolysiloxane (N) der allgemeinen Formel (1a) 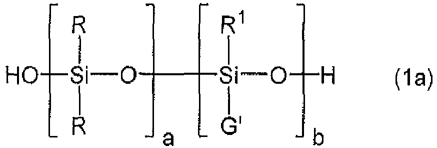 in der
- **G'**: eine Gruppe der allgemeinen Formel (2a) bedeutet 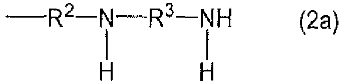
und **R, R¹, R², R³, R⁴**, **a** und **b** die vorstehenden Bedeutungen aufweisen,
mit acyclischen Carbonsäureanhydriden der allgemeinen Formel (3)

R⁵-C(=O)-O-C(=O)-R⁵ (3)

, in stöchiometrisch äquivalenter Menge zu den im Organopolysiloxan (N) vorhandenen Amingruppen in Substanz umgesetzt wird und nach erfolgter Acylierung sofort ein Neutralisationsmittel (B) zugegeben wird.

Das erhaltene Organopolysiloxan (O) ist damit überraschenderweise viskositätsstabil herstellbar.

Die bei beiden Verfahren gewählte Vorgehensweise, acyclische Carbonsäureanhydride der allgemeinen Formel (3) in stöchiometrisch äquivalenter Menge zu den in Organopolysiloxan (N) vorhandenen Amingruppen einzusetzen, hat ferner im Gegensatz zu dem in EP-A-161 888 verwendeten Überschuss an Acetylierungsmittel den Vorteil, dass ein Abdestillieren des Überschusses bei erhöhten Temperaturen nicht notwendig ist, was bei den beschriebenen Hydroxy-terminierten Organopolysiloxanen zwangsläufig zu einer Viskositätserhöhung führen würde.

Der Umsatz in "stöchiometrisch äquivalenter Menge zu den im Organopolysiloxan (N) vorhandenen Amingruppen" bedeutet, dass pro mol Stickstoffatom in der allgemeinen Formel (2a) 1 bis höchstens 1,1, vorzugsweise höchstens 1,05 mol, insbesondere höchstens 1,01 mol acyclisches Carbonsäureanhydrid der allgemeinen Formel (3) eingesetzt wird.

Der einwertige Kohlenwasserstoffrest **R** kann halogensubstituiert, linear, zyklisch, verzweigt, aromatisch, gesättigt oder ungesättigt sein. Vorzugsweise weist **R** 1 bis 6 Kohlenstoffatome auf, besonders bevorzugt sind lineare Alkylreste und Phenylreste. Bevorzugte Halogensubstituenten sind Fluor und Chlor. Besonders bevorzugte einwertige Kohlenwasserstoffreste **R** sind Methyl, Ethyl, Phenyl.

Der Alkylrest **R¹** wird vorzugsweise ausgewählt aus Methyl, Ethyl, n-Propyl, i-Propyl, Hydroxy, Methoxy, Ethoxy, n-Propoxy und i-Propoxy.

Vorzugsweise bedeuten die Reste **R², R³** einen Ethylen-, n-Propylen-, iso-Butylen- oder n-Butylenrest. Besonders bevorzugt als Rest **G'** ist der Rest -(CH₂)₃NH(CH₂)NH₂.

**R⁵** kann linear, zyklisch oder verzweigt sein. Vorzugsweise weist **R⁵** 1 bis 6 Kohlenstoffatome auf, besonders bevorzugt sind lineare Alkylreste, insbesondere Methyl, Ethyl, n-Propyl oder i-Propyl.

**a** bedeutet vorzugsweise ganzzahlige Werte von mindestens 200, besonders bevorzugt mindestens 300, insbesondere mindestens 500 und höchstens 1300, besonders bevorzugt höchstens 1100 und insbesondere höchstens 900.

**b** bedeutet vorzugsweise ganzzahlige Werte von höchstens 100, besonders bevorzugt höchstens 10, insbesondere höchstens 5.

Vorzugsweise werden **a** und **b** so gewählt, dass das Organopolysiloxan (0) eine Viskosität von mindestens 100, mehr bevorzugt mindestens 1000, besonders bevorzugt mindestens 5 000, insbesondere mindestens 15 000 mPas und höchstens 500 000, mehr bevorzugt höchstens 200 000, besonders bevorzugt höchstens 100 000, insbesondere höchstens 60 000 mPas aufweist.

Die Aminzahl des eingesetzten Organopolysiloxans (N) beträgt vorzugsweise mindestens 0,001, insbesondere mindestens 0,01 mmol/g und höchstens 5, besonders bevorzugt höchstens 1, ganz besonders bevorzugt höchstens 0,1, insbesondere höchstens 0,05 mmol/g.

Die Aminzahl bezeichnet die Menge an mol HCl, die zum Neutralisieren von 1 g Organopolysiloxan (N) erforderlich sind.

Zu Herstellung der wässrigen Emulsionen (W) können als Emulgatoren (E) alle bisher bekannten, ionischen und nichtionischen Emulgatoren sowohl einzeln als auch als Mischungen verschiedener Emulgatoren eingesetzt werden, mit denen auch bisher wässrige Dispersionen, insbesondere wässrige Emulsionen von Organopolysiloxanen hergestellt werden konnten. Ebenso können bekanntermassen anorganische Feststoffe als Emulgatoren (E) eingesetzt werden. Dies sind z.B. Kieselsäuren oder Bentonite wie in EP 1017745 A oder DE 19742759 A beschrieben.

Beispiele für anionische Emulgatoren sind:
1. Alkylsulfate, besonders solche mit einer Kettenlänge von 8 bis 18 C-Atomen, Alkyl- und Alkarylethersulfate mit 8 bis 18 C-Atomen im hydrophoben Rest und 1 bis 40 Ethylenoxid (EO)- bzw. Propylenoxid (PO)-Einheiten.
2. Sulfonate, besonders Alkylsulfonate mit 8 bis 18 C-Atomen, Alkylarylsulfonate mit 8 bis 18 C-Atomen, Tauride, Ester und Halbester der Sulfobernsteinsäure mit einwertigen Alkoholen oder Alkylphenolen mit 4 bis 15 C-Atomen; gegebenenfalls können diese Alkohole oder Alkylphenole auch mit 1 bis 40 EO-Einheiten ethoxyliert sein.
3. Alkali- und Ammoniumsalze von Carbonsäuren mit 8 bis 20 C-Atomen im Alkyl-, Aryl-, Alkaryl- oder Aralkylrest.
4. Phosphorsäureteilester und deren Alkali- und Ammoniumsalze, besonders Alkyl- und Alkarylphosphate mit 8 bis 20 C-Atomen im organischen Rest, Alkylether- bzw. Alkaryletherphosphate mit 8 bis 20 C-Atomen im Alkyl- bzw. Alkarylrest und 1 bis 40 EO-Einheiten.

Beispiele für nichtionische Emulgatoren sind:
5. Polyvinylalkohol, der noch 5 bis 50%, vorzugsweise 8 bis 20 Vinylacetateinheiten aufweist, mit einem Polymerisationsgrad von 500 bis 3000.
6. Alkylpolyglycolether, vorzugsweise solche mit 3 bis 40 EO-Einheiten und Alkylresten von 8 bis 20 C-Atomen.
7. Alkylarylpolyglycolether, vorzugsweise solche mit 5 bis 40 EO-Einheiten und 8 bis 20 C-Atomen in den Alkyl- und Arylresten.
8. Ethylenoxid/Propylenoxid(EO/PO)-Blockcopolymere, vorzugsweise solche mit 8 bis 40 EO- bzw. PO-Einheiten,
9. Additionsprodukte von Alkylaminen mit Alkylresten von 8 bis 22 C-Atomen mit Ethylenoxid oder Propylenoxid.
10. Fettsäuren mit 6 bis 24 C-Atomen.
11. Alkylpolyglykoside der allgemeinen Formel R*-O-ZO, worin R* einen linearen oder verzweigten, gesättigten oder ungesättigten Alkylrest mit im Mittel 8-24 C-Atomen und ZO einen Oligoglykosidrest mit im Mittel o = 1-10 Hexose- oder Pentoseeinheiten oder Gemischen davon bedeuten.
12. Naturstoffe und deren Derivate, wie Lecithin, Lanolin, Saponine, Cellulose; Cellulosealkylether und Carboxyalkylcellulosen, deren Alkylgruppen jeweils bis zu 4 Kohlenstoffatome besitzen.
13. Polare Gruppen, enthaltend insbesondere die Elemente O, N, C, S, P, Si, enthaltende lineare Organo(poly)siloxane, insbesondere solche mit Alkoxygruppen mit bis zu 24 C-Atomen und/oder bis zu 40 EO- und/oder PO-Gruppen.

Beispiele für kationische Emulgatoren sind:
14. Salze von primären, sekundären und tertiären Fettaminen mit 8 bis 24 C-Atomen mit Essigsäure, Schwefelsäure, Salzsäure und Phosphorsäuren.
15. Quarternäre Alkyl- und Alkylbenzolammoniumsalze, insbesondere solche, deren Alkylgruppen 6 bis 24 C-Atome besitzen, insbesondere die Halogenide, Sulfate, Phosphate und Acetate,
16. Alkylpyridinium-, Alkylimidazolinium- und Alkyloxazoliniumsalze, insbesondere solche, deren Alkylkette bis zu 18 C-Atome besitzt, speziell die Halogenide, Sulfate, Phosphate und Acetate.

Als Ampholytische Emulgatoren eignen sich besonders:
17. Langkettig substituierte Aminosäuren, wie N-Alkyl-di-(aminoethyl-)glycin oder N-Alkyl-2-aminopropionsäuresalze,
18. Betaine, wie N-(3-Acylamidopropyl)-N,N-dimethylammoniumsalze mit einem C₈-C₁₈-Acylrest und Alkylimidazolium-Betaine.

Bevorzugt als Emulgatoren sind nichtionische Emulgatoren, insbesondere die vorstehend unter 6. aufgeführten Alkylpolyglycolether und kationische Emulgatoren, insbesondere die vorstehend unter 15. aufgeführten quarternären Alkyl- und Alkylbenzolammoniumsalze. Der Bestandteil (E) kann aus einem der o.g. Emulgatoren oder aus einem Gemisch zweier oder mehrerer o.g. Emulgatoren bestehen, er kann in reiner Form oder als Lösungen eines oder mehrerer Emulgatoren in Wasser oder organischen Lösungsmitteln eingesetzt werden.

Gegenstand der Erfindung sind weiterhin wässrige Emulsionen (W) enthaltend 5 Gew.-% bis 70 Gew.-% der Hydroxy-terminierten Organopolysiloxane (O) der allgemeinen Formel (1) sowie 2 bis 150 Gew.% bezogen auf das Gewicht des Organopolysiloxanes (O) eines Emulgators (E).

Die wässrigen Emulsionen (W) bestehen aus einer diskontinuierlichen Ölphase, die das acylierte Organopolysiloxan (1) umfasst, den Emulgatoren und der kontinuierlichen Wasserphase (Öl-in-Wasser-Emulsion).

Die Gewichtsverhältnisse der diskontinuierlichen Ölphase und der kontinuierlichen Wasserphase können in weiten Bereichen variiert werden. In der Regel beträgt der Anteil der Ölphase 5 bis 70 Gew.%, vorzugsweise 10 bis 60 Gew.%, jeweils bezogen auf das Gesamtgewicht der Emulsion (W). Der Anteil an Emulgatoren liegt vorzugsweise im Bereich von 2 bis 150 Gew.%, insbesondere bis 50 Gew.%, jeweils bezogen auf das Gewicht der Ölphase.

Die bevorzugte mittlere Teilchengröße der diskontinuierlichen Ölphase beträgt weniger als 2 µm, insbesondere weniger als 1 µm. Besonders bevorzugt sind mittlere Teilchengrößen von höchstens 0,8 µm, insbesondere höchstens 0,5 µm.

Die Emulsion (W) kann für bestimmte Zwecke, beispielsweise für den Einsatz in kosmetischen Zusammensetzungen, neben den vorstehenden Bestandteilen Zusatzstoffe enthalten. Geeignete Zusatzstoffe sind beispielsweise Biozide, wie Fungizide, Bakterizide, Algicide und Mikrobicide, Verdickungsmittel, Frostschutzmittel, Antistatika, Farbstoffe, Flammschutzmittel und organische Weichmacher.

Emulgatoren (E1), die beim Verfahren A während der Acetylierung verwendet werden, müssen sich gegenüber den acyclischen Carbonsäureanhydriden im Wesentlichen inert verhalten. Unter dieser Prämisse werden sie vorzugsweise aus den vorstehend beschriebenen Emulgatoren (E) ausgewählt.

Bevorzugte Emulgatoren (E1) sind
(a) Alkylpolyglycolether, vorzugsweise solche mit 3 bis 40 EO-Einheiten und Alkylresten von 8 bis 20 C-Atomen.
(b) Alkylarylpolyglycolether, vorzugsweise solche mit 5 bis 40 EO-Einheiten und 8 bis 20 C-Atomen in den Alkyl- und Arylresten.
(c) Ethylenoxid/Propylenoxid (EO/PO)-Blockcopolymere, vorzugsweise solche mit 8 bis 40 EO- bzw. PO-Einheiten.
(d) Additionsprodukte von Alkylaminen mit Alkylresten von 8 bis 22 C-Atomen mit Ethylenoxid oder Propylenoxid.
oder kationische Emulgatoren wie
(e) Quarternäre Alkyl- und Alkylbenzolammoniumsalze, insbesondere solche, deren Alkylgruppen 6 bis 24 C-Atome besitzen, insbesondere die Halogenide, Sulfate, Phosphate und Acetate.
(f) Alkylpyridinium-, Alkylimidazolinium- und Alkyloxazoliniumsalze, insbesondere solche, deren Alkylkette bis zu 18 C-Atome besitzt, speziell die Halogenide, Sulfate, Phosphate und Acetate.

Besonders bevorzugt als Emulgatoren sind nichtionische Emulgatoren, insbesondere die vorstehend unter (a). aufgeführten. Der Bestandteil (E1) kann aus einem der o.g. Emulgatoren oder aus einem Gemisch zweier oder mehrerer o.g. Emulgatoren bestehen.

Neutralisationsmittel (B), die im Herstellungsverfahren B verwendet werden, sind alkalisch wirkende Salze wie (Erd)alkalimetallhydroxide, z.B. Natriumhydroxid, Kaliumhydroxid, Lithiumhydroxid, (Erd)alkalimetallcarbonate, z.B. Kaliumcarbonat, Natriumcarbonat, Lithiumcarbonat, (Erd)alkalisilanolate, z.B. Natriumtrimethylsilanolat, Lithiumtrimethylsilanolat, (Erd)alkalisiloxanolate, z.B. Lithiumsiloxanolat, (Erd)alkalialkanolate, z.B. Natriummethanolat, Natriumethanolat, Kalium-tert,-butanolat oder Ammoniumsalze, z.B. Ammoniumhydroxid bzw. organische Amine, z.B. Triethanolamin (TEA), Diethanolamin, Ethanolamin, Triethylamin, Isopropylamin,

Die Drucke für die Verfahren A und B betragen vorzugsweise mindestens 0,010, insbesondere mindestens 0,05 MPa und vorzugsweise höchstens 10, besonders bevorzugt höchstens 1 MPa. Insbesondere wird bei dem Druck gearbeitet, der am Produktionsstandort vorherrscht.

Die Temperatur beim Verfahren A beträgt vorzugsweise mindestens 0°C, besonders bevorzugt mindestens 10°C, insbesondere mindestens 20°C und vorzugsweise höchstens 100°C, besonders bevorzugt höchstens 80 °C und insbesondere höchstens 60°C.

Die Temperatur beim Verfahren B beträgt vorzugsweise mindestens 0°C, besonders bevorzugt mindestens 10°C, insbesondere mindestens 20°C und vorzugsweise höchstens 100°C, besonders bevorzugt höchstens 80 °C und insbesondere höchstens 60°C.

Das Organopolysiloxan (1) kann entweder durch Brechen der Emulsion auf beliebige Weise, z.B. durch Zugabe wasserlöslicher organischer Lösemittel, z.B. Methanol, Ethanol, Isopropanol, Aceton, oder durch Zugabe von Salzen, wie Natriumchlorid, oder durch Extraktion mit organischen Lösemitteln, z.B. n-Hexan, n-Heptan, Mischungen aus n-Hexan-Isopropanol, n-Hexan-Aceton praktisch emulgatorfrei gewonnen werden.

Alle vorstehenden Symbole der vorstehenden.Formeln weisen ihre Bedeutungen jeweils unabhängig voneinander auf. In allen Formeln ist das Siliciumatom vierwertig.

In den folgenden Beispielen sind, falls jeweils nicht anders angegeben, alle Mengen- und Prozentangaben auf das Gewicht bezogen, alle Drücke 0,10 MPa (abs.) und alle Temperaturen 20°C.

### Beispiel 1

### Erfindungsgemäße Acetylierung nach Verfahren B:

600 g eines Aminöls der allgemeinen Formel (1a) mit R = Methyl, R¹ = Methoxy, G' = 3-((2-Aminoethyl)amino)propyl und a und b derart gewählt, dass das Öl eine Viskosität von 14 100 mPas und eine Aminzahl von 0,032 mmol/g besitzt, wird auf 40°C erwärmt und mit 1,96 g (19,2 mmol; äquimolar zum Amin-Gehalt) Essigsäureanhydrid versetzt und anschließend für 30 min bei 40°C gerührt. Das Reaktionsgemisch wird mit 2,86 g (19,2 mmol) Triethanolamin versetzt, 30 min bei 40°C gerührt und anschließend auf Raumtemperatur abgekühlt, Das acetylierte Aminöl der allgemeinen Formel (1) hat eine Viskosität von 22 200 mPas. Nach Lagerung über 84 d bei Raumtemperatur ist die Viskosität auf 29 800 Pas angestiegen.

### Beispiel 1a

### Nicht-erfindungsgemäße Acetylierung:

600 g eines Aminöls der allgemeinen Formel (1a) mit R = Methyl, R¹ = Methoxy, G' = 3-((2-Aminoethyl)amino)propyl und a und b derart gewählt, dass das Öl eine Viskosität von 14 100 mPas und eine Aminzahl von 0,032 mmol/g besitzt, wird auf 40°C erwärmt und mit 1,96 g (19,2 mmol; äquimolar zum Amin-Gehalt) Essigsäureanhydrid versetzt und anschließend für 30 min bei 40°C gerührt. Nach Abkühlen auf Raumtemperatur hat das acetylierte Aminöl eine Viskosität von 24 600 mPas. Nach Lagerung über 84 d bei Raumtemperatur ist die Viskosität auf 117 200 Pas angestiegen,

### Emulsionsherstellung:

In einem Emulgiergerät (Fa. PC-Laborsystem) werden 5,26 Gew.% Isotridecylhexaethoxylat, käuflich erwerblich unter dem Handelsnamen Lutensol^{®} IT6 (Fa. BASF), 3,45 Gew.% einer wässrigen Lösung von Cetyltrimethylammoniumchlorid (29%ig, erwerblich unter dem Handelsnamen Genamin^{®} CTAC, Fa. Clariant) und 6,00 Gew.% entsalztes Wasser vorgelegt. Anschließend werden 55,00 Gew.% Organopolysiloxan der allgemeinen Formel (1) hinzudosiert, und unter hoher Scherung wird eine Voremulsion hergestellt. Diese wird durch portionsweise Zugabe von insgesamt 30,17 Gew.% vollentsalztem Wasser verdünnt. Durch Zugabe von 0,02 Gew.% Essigsäure wird ein pH-Wert von 3,5 eingestellt. Es resultiert eine milchig-weisse stabile Emulsion, die eine durchschnittliche Teilchengrösse von 0,19 µm aufweist.

### Beispiel 2

### Erfindungsgemäße Acetylierung nach Verfahren A:

In einem Emulgiergerät (Fa. PC-Laborsystem) werden 5,26 Gew.% Isotridecylhexaethoxylat, käuflich erwerblich unter dem Handelsnamen Imbentin-T/060 (Fa. Kolb) und 55,00 Gew.% eines Aminöls der allgemeinen Formel (1a) mit R = Methyl, R¹ = Methoxy, G' = 3-((2-Aminoethyl)amino)propyl und a und b derart gewählt, dass das Öl eine Viskosität von 18 000 mPas und eine Aminzahl von 0,022 mmol/g besitzt. 0,12 Gew.% Essigsäureanhydrid werden hinzugegeben und die Reaktionsmischung 15 min gerührt. Anschließend werden 3,45 Gew.% einer wässrigen Lösung von Cetyltrimethylammoniumchlorid (29%ig, erwerblich unter dem Handelsnamen Genamin^{®} CTAC, Fa. Clariant) und 6,00 Gew.% entsalztes Wasser hinzudosiert, und unter hoher Scherung wird eine Voremulsion hergestellt, die durch portionsweise Zugabe von insgesamt 30,17 Gew.% vollentsalztem Wasser verdünnt wird. Es resultiert eine milchig-weisse stabile Emulsion, die eine durchschnittliche Teilchengrösse von 0,33 µm aufweist.

Aus einem Teil der Emulsion wird das gewünschte Organopolysiloxan der allgemeinen Formel (1) mit Isopropanol/n-Hexan extrahiert. Mittels ¹H-NMR kann die nahezu vollständige Acetylierung durch die beiden H₃C-CO-Signale bei 1,87 ppm und 2,03 ppm nachgewiesen werden.

### Beispiel 3

### Erfindungsgemäße Acetylierung nach Verfahren A:

Die Acetylierung und Emulsionsherstellung des Beispiels 2 wird sinngemäß wiederholt mit dem Unterschied, dass neben entsalztem Wasser folgende Startmaterialien verwendet wurden: 3,45 Gew.% Isotridecylhexaethoxylat, käuflich erwerblich unter dem Handelsnamen Imbentin-T/060 (Fa. Kolb), 55,00 Gew.% eines Aminöls der allgemeinen Formel (1a) mit R = R¹ = Methyl, G' = 3-((2-Aminoethyl)amino)propyl und a und b derart gewählt, dass das Öl eine Viskosität von 25.260 mPas und eine Aminzahl von 0,026 mmol/g besitzt, 0,12 Gew.% Essigsäureanhydrid, 3,45 Gew.% einer wässrigen Lösung von Cetyltrimethylammoniumchlorid (29%ig, erwerblich unter dem Handelsnamen Genamin^{®} CTAC, Fa. Clariant), Es resultiert eine milchig-weisse stabile Emulsion, die eine durchschnittliche Teilchengrösse von 0,26 µm aufweist.

Aus einem Teil der Emulsion wird das gewünschte Organopolysiloxan der allgemeinen Formel (1) mit Isopropanol/n-Hexan extrahiert. Mittels ¹H-NMR kann die nahezu vollständige Acetylierung durch die beiden H₃C-CO-Signale bei 1,87 ppm und 2,03 ppm nachgewiesen werden.

### Beispiel 4

### Erfindungsgemäße Acetylierung nach Verfahren A:

Die Acetylierung und Emulsionsherstellung des Beispiels 2 wird sinngemäß wiederholt mit dem Unterschied, dass neben entsalztem Wasser folgende Startmaterialien verwendet wurden: 3,45 Gew.% Isotridecylhexaethoxylat, käuflich erwerblich unter dem Handelsnamen Imbentin-T/060 (Fa. Kolb), 55,00 Gew.% eines Aminöls der Struktur (1a) mit R = R¹ = Methyl, G' = 3-((2-Aminoethyl)amino)propyl und a und b derart gewählt, dass das Öl eine Viskosität von 22.350 mPas und eine Aminzahl von 0,026 mmol/g besitzt, 0,12 Gew.% Essigsäureanhydrid, 3,45 Gew.% einer wässrigen Lösung von Cetyltrimethylammoniumchlorid (29%ig, erwerblich unter dem Handelsnamen Genamin^{®} CTAC, Fa. Clariant). Es resultiert eine milchig-weisse stabile Emulsion, die eine durchschnittliche Teilchengrösse von 0,33 µm aufweist.

Aus einem Teil der Emulsion wird das gewünschte Organopolysiloxan der allgemeinen Formel (1) mit Isopropanol/n-Hexan extrahiert. Es besitzt eine Viskosität von 35 000 mPas (25°C); dies zeigt, dass durch die in-situ-Acetylierung nur ein mäßiger und damit beherrschbarer Ölviskositätsanstieg erfolgte. Mittels ¹H-NMR kann die nahezu vollständige Acetylierung durch die beiden H₃C-CO-Signale bei 1,87 ppm und 2,03 ppm nachgewiesen werden.

### Beispiel 5:

Verwendung der Emulsion von Beispiel 4 in einer Shampoo- resp. Conditioner-Formulierung:

| **Shampoo-Formulierung** | | |
|---|---|---|
| INCI name | Trade Name | Conc (%) |
| Aqua (Water VES) | Water | 4,49 |
| Guar Hydroxypropyltrimonium Chloride | N-Hance^{®} 3000 | 0,22 |
| Sodium Laureth Sulfate | Genapol^{®} LRO 26,5% | 78,87 |
| Glycol Distearate | Genapol^{®} PMS | 1,32 |
| PEG-150 Distearate | Eumulgin^{®} EO 33 | 0,22 |
| Emulsion aus Beispiel 4 | | 3,64 |
| Cocamidopropyl Betaine | Genagen^{®} CAB 818 30% | 11,07 |
| Methylchloroisothiazolinone und Methylisothiazolinone. | Kathon^{®} CG | 0,07 |
| Sodium Chloride | Sodium Chloride Solution (25%) | 0,10 |

| **Conditioner-Formulierung** | | |
|---|---|---|
| INCI name | Trade Name | Conc (%) |
| Aqua (Water VES) | Water | 87,40 |
| Citric Acid | Citric Acid | 0,20 |
| Hydroxyethylcellulose | Tylose^{®} H 4000 P2 | 1,20 |
| Tetrasodium EDTA | EDETA^{®} B Pulver | 0,20 |
| Polysorbate 80 | Tween^{®} 80 | 1,00 |
| Behentrimonium Chloride | Genamin^{®} KDMP | 1,76 |
| Cetyl Alcohol | Cetylalkohol | 1,00 |
| Stearamidopropyl Dimethylamine | Incromine^{®} SD | 0,50 |
| Stearyl Alcohol | Stearylalkohol | 3,00 |
| Emulsion aus Beispiel 4 | | 3,64 |
| Methylchloroisothiazolinone und Methylisothiazolinone. | Kathon^{®} CG | 0,10 |

## Patentansprüche

1. Hydroxy-terminierte Organopolysiloxane (O) der allgemeinen Formel (1), 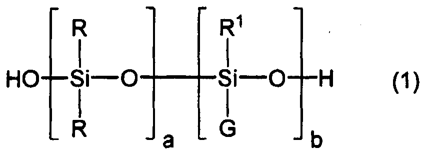 wobei
**R** einen einwertigen unsubstituierten oder halogensubstituierten Kohlenwasserstoffrest mit 1 bis 20 Kohlenstoffatomen,
**R¹** einen Rest **R**, -O**R⁴** oder -OH,
**R⁴** einen Alkylrest mit 1 bis 6 Kohlenstoffatomen,
**G** eine Gruppe der allgemeinen Formel (2), 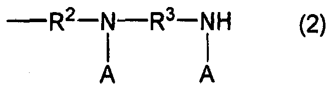
wobei
**R²**, **R³** einen zweiwertigen Kohlenwasserstoffrest mit 1 bis 6 C-Atomen, wobei nicht benachbarte -CH₂-Einheiten ersetzt sein können durch Einheiten, die ausgewählt werden aus -C(=O)-, -O-, und -S-,
**A** **R⁵**-C(=O)-,
**R⁵** Alkylrest mit 1 bis 20 Kohlenstoffatomen und
**a** ganzzahlige Werte von 100 bis 1500 und
**b** ganzzahlige Werte von mindestens 1 bedeuten.

2. Organopolysiloxane (O) gemäß Anspruch 1, bei denen **R** 1 bis 6 Kohlenstoffatome aufweist.

3. Organopolysiloxane (O) gemäß Anspruch 1 oder 2, bei denen die Reste **R², R³** ausgewählt werden aus Ethylen-, n-Propylen-, iso-Butylen- oder n-Butylenrest.

4. Organopolysiloxane (O) gemäß Anspruch 1 bis 3, bei denen **R⁵** lineare Alkylreste mit 1 bis 6 Kohlenstoffatomen bedeutet.

5. Wässrige Emulsionen (W) enthaltend 5 Gew.-% bis 70 Gew.-% der Hydroxy-terminierten Organopolysiloxane (O) der allgemeinen Formel (1) gemäß Anspruch 1 bis 4 sowie 1 bis 150 Gew.% bezogen auf das Gewicht des Organopolysiloxanes (O) eines Emulgators (E).

6. Verwendung der Emulsionen (W) gemäß Anspruch 5 in Zusammensetzungen für die Kosmetik.

7. Verfahren A zur Herstellung der Hydroxy-terminierten Organopolysiloxane (O) gemäß Anspruch 1 bis 4, bei dem Hydroxy-terminierte Organopolysiloxane (N) der allgemeinen Formel (1a) 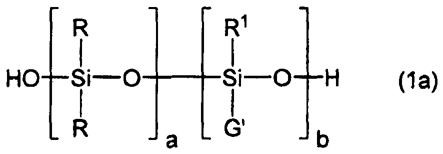 in der
**G'** eine Gruppe der allgemeinen Formel (2a) bedeutet 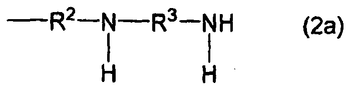
und **R, R¹**, **R², R³, R⁴ a** und **b** die in Anspruch 1 angegebenen Bedeutungen aufweisen,
mit acyclischen Carbonsäureanhydriden der allgemeinen Formel (3)
R⁵-C(=O)-O-C(=O)-R⁵ (3)
,
wobei **R⁵** die in Anspruch 1 angegebene Bedeutung aufweist, in stöchiometrisch äquivalenter Menge zu den im Organopolysiloxan (N) vorhandenen Amingruppen in Gegenwart von einem oder verschiedenen Emulgatoren (E1) umgesetzt wird.

8. Verfahren B zur Herstellung der Hydroxy-terminierten Organopolysiloxane (O) gemäß Anspruch 1 bis 4, bei dem Hydroxy-terminierte Organopolysiloxane (N) der allgemeinen Formel (1a) 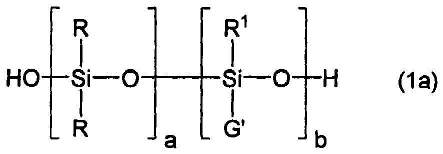 in der
**G'** eine Gruppe der allgemeinen Formel (2a) bedeutet 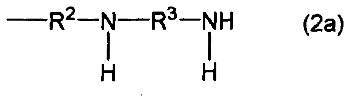
und **R, R¹, R², R³, R⁴, a** und **b** die vorstehenden Bedeutungen aufweisen,
mit acyclischen Carbonsäureanhydriden der allgemeinen Formel (3)
R⁵-C(=O)-O-C(=O)-R⁵ (3)
,
wobei **R⁵** die in Anspruch 1 angegebene Bedeutung aufweist, in stöchiometrisch äquivalenter Menge zu den im Organopolysiloxan (N) vorhandenen Amingruppen in Substanz umgesetzt wird, und nach erfolgter Acylierung sofort ein Neutralisationsmittel (B) zugegeben wird.

## Claims

1. Hydroxy-terminated organopolysiloxanes (O) of the general formula (1), 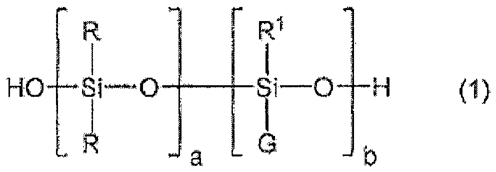 where
**R** is a monovalent unsubstituted or halogen-substituted hydrocarbon radical having 1 to 20 carbon atoms,
**R¹** is a radical **R, -OR⁴** or -OH,
**R⁴** is an alkyl radical having 1 to 6 carbon atoms,
**G** is a group of the general formula (2), 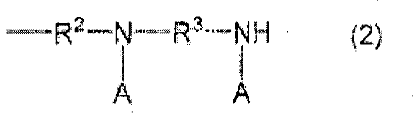
where
**R², R³** are a divalent hydrocarbon radical having 1 to 6 carbon atoms, where nonadjacent -CH₂ units can be replaced by units which are selected from -C(=O)-, -O- and -S-,
**A** is **R⁵**-C(=O)-,
**R⁵** is an alkyl radical having 1 to 20 carbon atoms and
**a** is integer values from 100 to 1500 and
**b** is integer values of at least 1.

2. Organopolysiloxanes (O) according to Claim 1, in which **R** has 1 to 6 carbon atoms.

3. Organopolysiloxanes (O) according to Claim 1 or 2, in which the radicals **R², R³** are selected from ethylene, n-propylene, isobutylene or n-butylene radical.

4. Organopolysiloxanes (O) according to Claims 1 to 3, in which **R⁵** is linear alkyl radicals having 1 to 6 carbon atoms.

5. Aqueous emulsions (W) comprising 5% by weight to 70% by weight of the hydroxy-terminated organopolysiloxanes (O) of the general formula (1) according to Claims 1 to 4, and 1 to 150% by weight, based on the weight of the organopolysiloxane (O), of an emulsifier (E).

6. Use of the emulsions (W) according to Claim 5 in compositions for cosmetics.

7. Process A for preparing the hydroxy-terminated organopolysiloxanes (O) according to Claims 1 to 4, in which hydroxy-terminated organopolysiloxanes (N) of the general formula (1a) 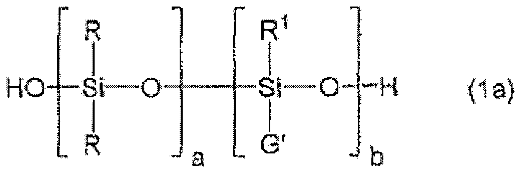 in which
**G'** is a group of the general formula (2a) 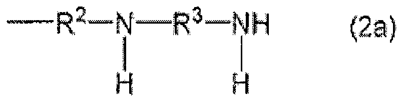
and **R**, **R¹**, **R²**, **R³**, **R⁴**, **a** and **b** have the meanings given in Claim 1,
are reacted with acyclic carboxylic anhydrides of the general formula (3)
**R⁵**-C(=O)-O-C(=O)-**R⁵** (3)
where R⁵ has the meaning given in Claim 1,
in a stoichiometrically equivalent amount to the amine groups present in the organopolysiloxane (N) in the presence of one or different emulsifiers (E1).

8. Process B for preparing the hydroxy-terminated organopolysiloxanes (O) according to Claims 1 to 4, in which hydroxy-terminated organopolysiloxanes (N) of the general formula (1a) 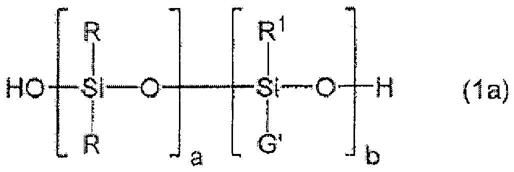 in which
**G'** is a group of the general formula (2a) 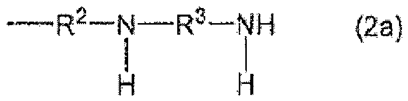
and **R**, **R¹**, **R²**, **R³**, **R⁴**, **a** and **b** have the aforementioned meanings,
are reacted with acyclic carboxylic anhydrides of the general formula (3)
**R⁵**-C(=O)-O-C(=O)-**R⁵** (3)
where R⁵ has the meaning given in Claim 1,
in a stoichiometrically equivalent amount to the amine groups present in the organopolysiloxane (N) without dilution, and after the acylation has taken place a neutralizing agent (B) is added immediately.

## Revendications

1. Organopolysiloxanes (O) à terminaison hydroxy, de formule générale (1), 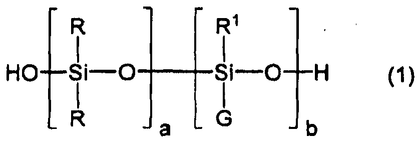 dans laquelle
**R** représente un radical hydrocarboné monovalent non substitué ou substitué par halogène, ayant de 1 à 20 atomes de carbone,
**R¹** représente un radical **R,** -O**R⁴** ou -OH,
**R⁴** représente un radical alkyle ayant de 1 à 6 atomes de carbone,
**G** représente un groupe de formule générale (2), 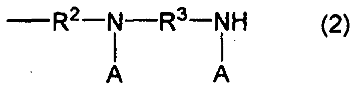
où
**R², R³** représentent un radical hydrocarboné divalent ayant de 1 à 6 atomes de carbone, dans lequel des unités -CH₂- non contiguës peuvent être remplacées par des unités qui sont choisies parmi -C(=O)-, -O- et -S-,
**A** représente **R⁵**-C(=O)-,
**R⁵** représente un radical alkyle ayant de 1 à 20 atomes de carbone et
**a** représente des nombres entiers valant de 100 à 1 500 et
**b** représente des nombres entiers valant au moins 1.

2. Organopolysiloxanes (O) selon la revendication 1, dans lesquels **R** comporte de 1 à 6 atomes de carbone.

3. Organopolysiloxanes (O) selon la revendication 1 ou 2, dans lesquels les radicaux **R², R³** sont choisis parmi le radical éthylène, n-propylène, isobutylène ou n-butylène.

4. Organopolysiloxanes (O) selon l'une quelconque des revendications 1 à 3, dans lesquels **R⁵** représente des radicaux alkyle linéaires ayant de 1 à 6 atomes de carbone.

5. Émulsions aqueuses (W) contenant 5 % en poids à 70 % en poids des organopolysiloxanes (O) à terminaison hydroxy, de formule générale (1) selon l'une quelconque des revendications 1 à 4, ainsi que 1 à 150 % en poids, par rapport au poids de l'organopolysiloxane (O), d'un émulsifiant (E).

6. Utilisation des émulsions (W) selon la revendication 5, dans des compositions pour la cosmétique.

7. Procédé A pour la préparation des organopolysiloxanes (O) à terminaison hydroxy, selon l'une quelconque des revendications 1 à 4, dans lequel on fait réagir des organopolysiloxanes (N) à terminaison hydroxy de formule générale (1a) 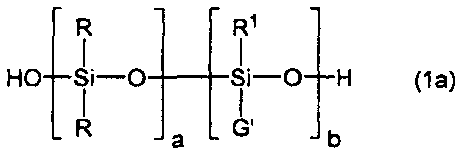 dans laquelle
**G'** représente un groupe de formule générale (2a) 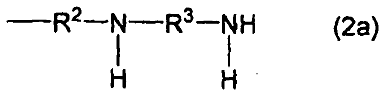
et **R, R¹, R², R³, R⁴, a** et **b** ont les significations indiquées dans la revendication 1,
avec des anhydrides d'acides carboxyliques acycliques de formule générale (3)
R⁵-C(=O)-O-C(=O)-R⁵ (3),
dans laquelle **R⁵** a la signification indiquée dans la revendication 1,
en quantité stoechiométriquement équivalente par rapport aux groupes amino présents dans l'organopolysiloxane (N)
en présence d'un ou de divers émulsifiants (E1).

8. Procédé B pour la préparation des organopolysiloxanes (O) à terminaison hydroxy, selon l'une quelconque des revendications 1 à 4, dans lequel on fait réagir tels quels des organopolysiloxanes (N) à terminaison hydroxy, de formule générale (1a) 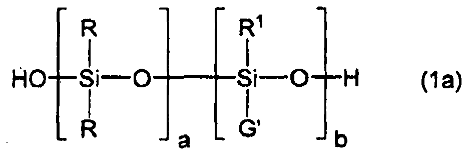 dans laquelle
**G'** représente un groupe de formule générale (2a) 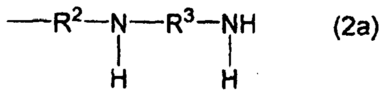
et **R, R¹, R², R³, R⁴, a** et **b** ont les significations précédentes,
avec des anhydrides d'acides carbocycliques acycliques de formule générale (3)
R⁵-C(=O)-O-C(=O)-R⁵ (3),
dans laquelle **R⁵** a la signification indiquée dans la revendication 1,
en quantité stoechiométriquement équivalente par rapport aux groupes amino présents dans l'organopolysiloxane (N), et une fois effectuée l'acylation on ajoute immédiatement un agent de neutralisation (B).
